# EUROPÄISCHE PATENTSCHRIFT

(11) **EP 0 932 599 B1**
(45) Veröffentlichungstag und Bekanntmachung des Hinweises auf die Patenterteilung: **22.08.2001**
(21) Anmeldenummer: 97911197.8
(22) Anmeldetag: 08.10.1997
(51) Int. Cl.: C07C 303/14, C07C 309/04

(54) **VERFAHREN ZUR HERSTELLUNG VON METHANSULFONSÄURE**
PROCESS TO MANUFACTURE METHANE SULFONIC ACID
PROCEDE DE FABRICATION D'ACIDE METHANESULFONIQUE

(30) Priorität: 09.10.1996 DE 19641483
(43) Veröffentlichungstag der Anmeldung: 04.08.1999
(73) Patentinhaber: BASF AKTIENGESELLSCHAFT, 67056 Ludwigshafen (DE)
(72) Erfinder: EIERMANN, Matthias, D-67117 Limburgerhof (DE); PAPKALLA, Thomas, D-68165 Mannheim (DE)
(86) Internationale Anmeldenummer: EP9705536
(87) Internationale Veröffentlichungsnummer: WO9815527

(56) Entgegenhaltungen:
- TETRAHEDRON LETTERS, Nr.27, 1966, OXFORD, GB Seiten 3095 - 3103, XP002054189 F. ASINGER, ET AL. 'Die Isomerenbildung bei der photochemischen Sulfochlorierung und Sulfoxydation von Carbonsäuren und Carbonsäurederivaten' in der Anmeldung erwähnt

## Beschreibung

Die vorliegende Erfindung betrifft ein verbessertes Verfahren zur Herstellung von Methansulfonsäure aus einer Essigsäure, Schwefeldioxid und Sauerstoff enthaltenden Mischung durch Bestrahlung mit Licht.

Methansulfonsäure ist als einfachster Vertreter der Klasse der Alkansulfonsäuren für eine Vielzahl von Anwendungen wie die galvanische Herstellung von Metallüberzügen oder aber auch als Veresterungskatalysator von großem technischen Nutzen.

Die gebräuchlichsten Herstellungsverfahren für Methansulfonsäure sind dabei die Oxidation von Methylmercaptan oder Dimethyldisulfid mit Sauerstoff oder mit Chlor zum Methansulfonylchlorid mit anschließender Hydrolyse. Alle diese Verfahren sind aufgrund ihrer Einsatzstoffe, die aus Schwefelwasserstoff gebildet werden, mit einem Toxizitäts- und Geruchsproblem behaftet, das nur mit großem technischen Aufwand beherrschbar ist.

In der DE 907 053 ist die Bestrahlung von Carbonsäuren in Gegenwart von Luft und Schwefeldioxid beschrieben. Die Reaktionsprodukte sind die entsprechenden β-Sulfocarbonsäuren.

Anders verläuft hingegen die Bestrahlung von Essigsäure bei Raumtemperatur in Gegenwart von Luft und Schwefeldioxid, wie in Tetrahedron Lett. 1966, 3095 beschrieben. Bei der Umsetzung wird Methansulfonsäure sowie zu 60 %, bezogen auf die gebildete Methansulfonsäure, auch Schwefelsäure erhalten. Ein technisches Verfahren mit einem derart hohen Nebenproduktanfall ist jedoch unwirtschaftlich.

Daher war es Aufgabe der vorliegenden Erfindung, ein wirtschaftliches Verfahren zur Verfügung zu stellen, nach dem die Methansulfonsäure in guter Ausbeute anfällt und die Schwefelsäure zu höchstens 50 %, bezogen auf die Methansulfonsäure, anfällt.

Demgemäß wurde ein Verfahren zur Herstellung von Methansulfonsäure durch Bestrahlung einer Essigsäure, Schwefeldioxid und Sauerstoff enthaltenden Mischung mit Licht gefunden, welches dadurch gekennzeichnet ist, daß man mit einer kumulierten Strahlungsdichte im Bereich von 240 bis 320 nm an der Lichteintrittsfläche des Lichts in die Reaktionsmischung im Mittel von 0,1 bis 50 mMol Quanten/cm²h bestrahlt.

Als Lampen werden bevorzugt solche eingesetzt, die Licht im Bereich von 240 bis 320 nm emittieren, wie Quecksilberniederdrucklampen, vorzugsweise Quecksilberhoch- und -mitteldrucklampen in reiner oder dotierter Ausführung, die handelsüblich sind und deren Glühleistung 125 Watt bis 60 kW beträgt. Ebenso geeignet sind Excimerlampen, die bevorzugt im Wellenlängenbereich von 240 bis 320 nm verwendet werden, in dem Schwefeldioxid eine starke Absorption aufweist.

Weiterhin sind Halogenglühlampen, Gasentladungslampen oder Leuchtstoffröhren als Lichtquellen geeignet.

Bevorzugt beträgt die kumulierte Strahlungsdichte im Bereich von 240 bis 320 nm an der Lichteintrittsfläche des Lichts in die Reaktionsmischung im Mittel nicht mehr als 10 mMol Quanten/cm²h und hat bei Strahlungsdichten bis zu 5 mMol Quanten/cm²h einen optimalen Wirkungsgrad.

Durch Kenntnis des Quantenstroms der Lampe, der in der Regel vom Hersteller angegeben wird, ist es leicht möglich, einen Reaktor mit geeigneter Bestrahlungsfläche abhängig von der Lampenleistung und der gewünschten Umsatzmenge zu wählen.

So hat eine herkömmliche 150 W Quecksilberhochdrucklampe im Wellenlängenbereich von 240-320 nm einen Quantenstrom von 0,128 Mol Quanten/h und eine 700 W Lampe von 0,6 Mol Quanten/h.

Es ist allgemein bekannt, bei Bestrahlungsreaktionen für eine gute Durchmischung, insbesondere in der Eintrittszone des Lichtes in das Reaktionsgemisch, zu sorgen.

Eine gute Durchmischung wird beispielsweise erreicht, indem man turbulente Wandströmung oder hohe Wandgeschwindigkeiten der Flüssigkeit erzeugt. Dies kann durch im Bestrahlungsbereich gekrümmte Reaktorrohre, beispielsweise in Form einer Schleife oder Wendel, erreicht werden, wenn die Reaktionsgase durch die Flüssigkeit perlen. Generell kann eine wirkungsvolle Durchmischung erzielt werden, indem man die Reaktionsgase als Strom feinverteilter Gasblasen durch die Reaktionsmischung leitet. Dieser Effekt kann ferner erreicht oder zusätzlich noch unterstützt werden, wenn die Flüssigkeit ihrerseits durch den Reaktor befördert wird. Vorteilhaft ist auch die Verwendung eines Gasrückvermischungsrührers (Hohlwellenrührer).

Bevorzugte Reaktortypen für das Verfahren sind beispielsweise Rohrreaktoren, Rohrbündelreaktoren, Schlaufenreaktoren oder Gleichstrom-Füllkörperkolonnen, die dem Fachmann allgemein bekannt sind.

In einer besonders bevorzugten Ausführungsform wird das Reaktionsgemisch im Kreislauf bewegt, beispielsweise durch Pumpen oder durch Rührer, wie es im Schlaufenreaktor der Fall ist. Vorteilhaft durchströmt die Reaktionsmischung den Reaktor mit Durchflußgeschwindigkeiten von 0,01 m/s bis 1 m/s.

Prinzipiell sind die verschiedenen Schlaufenreaktortypen, wie sie in "Chemische Reaktionstechnik, Lehrbuch der technischen Chemie", Bd. 1, Thieme Verlag, Stuttgart, New York 1992, S. 257 - 262), beschrieben sind, geeignet. Die Flüssigkeit kann dabei in einem inneren oder äußeren Kreislauf geführt werden.

Das erfindungsgemäße Verfahren kann sowohl kontinuierlich wie auch diskontinuierlich geführt werden.

Es ist vorteilhaft, wenn die Schichtdicke der zu bestrahlenden Flüssigkeit ein mehrfaches der Eindringtiefe der relevanten Strahlung beträgt. Bevorzugt beträgt die Schichtdicke der zu bestrahlenden Flüssigkeitszone mindestens 1 cm, besonders bevorzugt mindestens 5 cm. Ebenso ist es aus Wirtschaftlichkeitsgründen zur Verringerung des Hold-ups sinnvoll, die Schichtdicke der zu bestrahlenden Flüssigkeit nicht größer als 150 cm, bevorzugt nicht größer als 50 cm, zu wählen.

In technischen Apparaturen ordnet man die Lichtquelle vor entsprechend lichtdurchlässigen Fenstern, wie Quarzglas, der Reaktionsgefäße oder bevorzugt als Tauchlampen zentral oder radial in den Reaktionsräumen an.

Dabei ist es durchaus möglich, mehr als eine Lampe zu verwenden, wenn eine größere Stoffmenge umgesetzt werden soll. Es ist prinzipiell auch möglich, mehrere Lampen in einem Reaktor anzubringen. (Ullmanns Encyklopädie der technischen Chemie, 4. Aufl., Bd. 3, Verlag Chemie, Weinheim, 1973, S. 305 - 319; Ullmann's encyclopedia of industrial Chemistry, Vol A 19, 5. ed, VCH, Weinheim, 1991, p. 573 to 586.)

In einer vorteilhaften Ausführungsform wählt man einen Schlaufenreaktor, bei dem das Reaktionsgemisch an mehreren Lampen nacheinander vorbeigeführt wird. Aus Raumgründen werden die Lampen vorteilhaft zueinander parallel angeordnet, so daß ein sogenannter Schlangenreaktor vorliegt. Bevorzugt ist in diesem Fall die Verwendung von Tauchlampen, doch ist es ebenso möglich, durch geeignete Anordnung die Reaktionsmischung auch zwischen den einzelnen Lampen vorbeizuführen. Verwendet man ein wendelförmiges Reaktorrohr, so plaziert man die Lampe bevorzugt in der Wickelachse der Wendel.

Besonders vorteilhaft ist die Verwendung eines Schlaufenreaktors mit innenliegender, konzentrisch angeordneter Tauchlampe, oder eines Rohrbündelreaktors, bei welchem die einzelnen Rohre in Form eines Rings kreisförmig um eine Lichtquelle herum angeordnet sind und jedes wahlweise in Parallel- oder Serienschaltung von dem Reaktionsgemisch durchströmt wird. Im letztgenannten Fall ist es vorteilhaft, die einzelnen Rohre des Rohrbündels zur Lichtquelle hin abgeflacht auszuführen, um Strahlungsverluste durch Beugung oder Reflexion zu minimieren.

Der Reaktionsmischung können zur besseren Nutzung der von der Strahlungsquelle emittierten Strahlung oder aber auch zur Förderung oder Hemmung einzelner Teilschritte, der im Reaktionsgemisch ablaufenden Reaktionen, und damit letztlich zur Steigerung der Ausbeute Hilfsstoffe zugesetzt werden.

In einer bevorzugten Ausführungsform werden der Mischung Sensibilisatoren oder Photoinitiatoren als Hilfsstoffe zugefügt, die die ausschließliche oder zusätzliche Nutzung von langwelliger Strahlung ermöglichen. Herkömmliche Photoinitiatoren sind z.B.:
- thermische Startsysteme, wie Wasserstoffperoxid, Peroxide oder Azoisobutyronitril,
- Ketone, wie Acetophenon, Benzophenon oder Benzanthron,
- Acyloine, wie Benzoin-Derivate,
- α-Diketone wie Diacetyl, Phenanthrenchinon oder Benzil,
- Chinone, wie Anthrachinon-Derivate,
- Schwefelverbindungen, wie Diphenyldisulfid oder Tetramethylthiuramdisulfid,
- Halogenverbindungen, wie Chlor, Bromtrichlormethan, Bromoform oder Styroldibromid,
- Metallcarbonyle und Perchlorverbindungen wie Mangancarbonyl und org. Halogenverbindung,
sowie Hexaarylbisimidazol, fettsaure Iodsalze, α-Diketonmonooximester, Triphenylphosphin, organische Sulfinsäuren und Farbstoffe, Bis- (arylsulfonyl)-diazomethan, Buntesalze oder Uranylsalze.

Weiterhin bevorzugte Hilfsstoffe sind Stickoxide, Chloroform sowie die Hydroxide, die anorganischen oder organischen Salze von Kupfer oder Kobalt, wie Kupfer- oder Kobalthydroxid, Kupfer- oder Kobaltsulfat, Kupfer- oder Kobaltsulfit, Kupfer- oder Kobaltcarbonat, Kupfer- oder Kobalthydrogencarbonat, Kupfer- oder Kobaltmethansulfonat oder bevorzugt Kupfer- oder Kobaltacetat.

In der Regel werden die Hilfsstoffe in einer Konzentration von 10⁻² bis 10 Gew.-%, vorzugsweise 10⁻¹ bis 5 Gew.-%, jeweils bezogen auf Essigsäure, eingesetzt.

Für die Umsetzung ist Essigsäure in technisch verfügbaren Reinheiten geeignet.

Die Umsetzung der Essigsäure mit Schwefeldioxid und Sauerstoff unter Belichtung führt man so durch, daß man für eine möglichst homogene Mischung der Gase mit der Essigsäure sorgt. So läßt sich durch bekannte Maßnahmen, z.B. durch Eindüsen, eine feine Verteilung der Reaktionsgase herbeiführen.

Das Verhältnis von Schwefeldioxid zu Sauerstoff kann dabei variieren. In der Regel werden äquimolare Gasmengen durch die Reaktionsmischung gegast. Ebenso ist jedoch ein bis zu 10 molarer Überschuß eines der Gase möglich. Generell ist es vorteilhaft, annähernd im erfindungsgemäßen Temperaturbereich gasgesättigte Essigsäure zu verwenden.

In einer bevorzugten Ausführungsform werden 0,01 bis 10 mol Schwefeldioxid pro Mol Essigsäure kontinuierlich über den Bestrahlungszeitraum eingegast. Gleiches gilt für den Sauerstoff. Die beiden Gase können als Gemisch oder getrennt eingeleitet werden.

Schwefeldioxid kann dabei in reiner Form flüssig oder gasförmig oder als Lösung in Essigsäure oder einem geeigneten Lösungsmittel, wie Wasser, der Reaktion zugeführt werden.

Man kann Schwefeldioxid im Gasgemisch mit Sauerstoff oder einer Sauerstoff enthaltenden Gasmischung, wie Luft, der Reaktion zusetzen. Die Verwendung eines Schwefeldioxid/Luft-Gemisches hat niedrigere Partialdrücke des Schwefeldioxids und des Sauerstoffs zur Folge, was jedoch auf das erfindungsgemäße Verfahren keinen spürbaren Einfluß hat, da sich in der Regel Essigsäure, Schwefeldioxid und Sauerstoff bezogen auf die effektive Strahlungsmenge im Überschuß befinden.

Bevorzugt werden Schwefeldioxid und Sauerstoff oder Schwefeldioxid und Luft gemeinsam eingeleitet.

Zur Dispergierung der Gase oder der Gasmischung in der Flüssigkeit können allgemein gebräuchliche Verteiler wie Sinterplatten, Lochplatten, Siebböden und Düsen, bevorzugt Glasfritten oder Ringdüsen, verwendet werden.

Die Gasabscheidung findet auf allgemein übliche Weise statt, nachdem das Gas die Bestrahlungszone durchlaufen hat.

Das erfindungsgemäße Verfahren ist von Druck weitgehend unabhängig. Man kann bei erhöhtem Druck arbeiten, beispielsweise bis zu 20 bar bevorzugt bis zur 15 bar. Es ist ebenfalls möglich, einen geringen Unterdruck bis beispielsweise 500 mbar zu wählen. Unterdruck verringert die Konzentration des in der Essigsäure gelösten Schwefeldioxids, so daß eine zu geringe SO₂-Konzentration den Umsatz herabsetzt. Besonders bevorzugt ist der Druckbereich von 1 bis 10 bar.

Das erfindungsgemäße Verfahren läßt sich vorteilhaft ab Temperaturen von 0°C, bevorzugt 20°C, insbesondere 50°C durchführen. Oberhalb 160°C überwiegen Nebenreaktionen, so daß die Bestrahlung bevorzugt bei Temperaturen bis 140°C, besonders bevorzugt bis 120°C, durchgeführt wird. Die Mischung kann beispielsweise, wie allgemein bekannt, mit Hilfe eines lichtquellenseitigen Doppelmantels und einem UV-durchlässigen Medium wie Wasser thermostatisiert werden.

Die Reaktion kann in inerten Verdünnungsmitteln durchgeführt werden, wobei "inert" die Tatsache mit umfaßt, daß das Verdünnungsmittel im genutzten Wellenlängenbereich keine nennenswerte Eigenabsorption hat.

Bevorzugt wird die Reaktion jedoch ohne Verdünnungsmittel durchgeführt.

Die Bestrahlungsdauer ist abhängig von der verwendeten Essigsäuremenge, Temperatur, Druck sowie der Strahlungsleistung der Lampen. Beispielsweise bestrahlt man mit einer 150 Watt Quecksilberhochdrucklampe mit einer Lichtemission von 0,128 mol Quanten/h kumuliert von 240 bis 320 nm 1 Liter Reaktionslösung über einen Zeitraum von 15 Minuten bis 20 Stunden, vorzugsweise 1 bis 10 Stunden.

Es kann aufgrund von Nebenproduktbildung vorteilhaft sein, die Reaktion nur bis zu einem Teilumsatz zu führen. Bevorzugt wird die Reaktion bis zu einem 20 Gew.-%igen, insbesondere einem 15 Gew.-%igen, Methansulfonsäuregehalt am Gesamtaustrag durchgeführt.

Die Isolierung des Produktes geschieht auf allgemein bekannte Weise, in der Regel durch Destillation. Bevorzugt wird in einer ersten Destillationsstufe Essigsäure abdestilliert und aus dem verbleibenden Sumpf in einer zweiten Stufe die Methansulfonsäure abdestilliert. Die Destillationen können jeweils diskontinuierlich oder kontinuierlich, bevorzugt beide kontinuierlich, durchgeführt werden. Die Destillationen können bei Drücken von 0,01 mbar bis 1 bar, bevorzugt zwischen 0,1 und 100 mbar, durchgeführt werden. Die bei unvollständiger Umsetzung derart abgetrennte Essigsäure, gegebenenfalls im Gemisch mit Spuren von Methansulfonsäure und/oder Schwefelsäure, kann so erneut eingesetzt werden. Ferner wird die Aufarbeitung bevorzugt so ausgelegt, daß die als Nebenprodukt gebildete Schwefelsäure in Schwefelsäurespaltanlagen verwertet werden kann.

Das erfindungsgemäße Verfahren kann beispielsweise so durchgeführt werden, daß die Essigsäure in einen Quarzglas-Rohrwendelreaktor, in dessen Wickelachse der Rohrwendel sich eine Quecksilberhochdrucklampe befindet, bestrahlt wird. Anfang und Ende der Rohrwendel sind über eine außenliegende Leitung verbunden, so daß die Reaktionsmischung mittels einer Pumpe im Kreis gefördert wird. In derselben Strömungsrichtung wie die Flüssigkeit, werden die beiden Gase kontinuierlich eingegast.

In einer bevorzugten Ausführungsform wird anstelle des Rohrwendelreaktors ein rohrförmiger Reaktor mit einer Quecksilberhochdruck-Tauchlampe bei ansonsten gleicher Anordnung gewählt.
Die nachfolgenden Beispiele sollen das erfindungsgemäße Verfahren näher erläutern.

Das erfindungsgemäße Verfahren zeichnet sich durch gute Ausbeute sowie einem hohen Produktgehalt von Methansulfonsäure und damit einer guten Selektivität aus.

### Beispiel 1

- Apparatur:: Ein Quarzglas-Rohrwendel mit einem Rohrinnendurchmesser von 1 cm und einer gewendelten Rohrlänge von 1,6 m und einem Wendelinnendurchmesser von 7 cm ist an ihren Enden über eine äußere Leitung mit zwischengeschalteter Pumpe verbunden. In der Wickelachse der Rohrwendel ist eine 150 Watt Quecksilberhochdruckdampflampe angebracht.

In diesen Reaktor wurden aufsteigend 300 g Essigsäure mit einem Durchfluß von 80 l/h im Kreis gepumpt. Am Einlauf der Rohrwendel wurden 10 Normliter Luft/h und 10 Normliter Schwefeldioxid/h gemeinsam kontinuierlich eingegast und hinter dem Auslauf über einen Gas/Flüssig-Abscheider abgetrennt. Das Reaktionsgemisch wurde 6,5 h bei 90°C bestrahlt und ausschließend destillativ aufgearbeitet. Die Analytik erfolgte mittels quantitativer Ionenchromatographie.

In der nachfolgenden Tabelle 1 sind Reaktionsbedingungen und Versuchsergebnisse von Beispiel 1 sowie den analog durchgeführten Beispielen 2 und 3 zusammengefaßt.

**Tabelle 1:**

| Reaktionsbedingungen und Ergebnis der photochemischen Herstellung von Methansulfonsäure in einem Rohrwendelreaktor | | | | | | |
|---|---|---|---|---|---|---|
| Bsp. | T [°C] | Essigsäure [g] | Reaktionszusatz [Gew.-%] | MSS [g] | Gewichtsverhältnis H₂SO₄/MSS | Rate MSS [g/kWh] |
| 1 | 90 | 300 | - | 20,8 | 35/100 | 15 |
| 2 | 90 | 330 | 0,1 % Cu(OCOCH₃)₂ | 13,9 | 23/100 | 14 |
| 3 | 90 | 314 | 5 % CHCl₃ | 20,6 | 31/100 | 21 |
| MSS = Methansulfonsäure. | | | | | | |

Ein analog Beispiel 1 durchgeführter Versuch bei einer Temperatur von 60°C und 330 g Essigsäure als Ausgangsverbindung ergab 18,4 g Methansulfonsäure. Ferner ergab eine analog Beispiel 1 durchgeführte Bestrahlung bei einer Temperatur von 30°C 14,0 g Methansulfonsäure.

### Beispiel 4 (Vergleich)

In einem zylindrischen Rührkolben (Höhe 14 cm, Durchmesser 4,5 cm) aus Quarzglas, neben dem sich im Abstand von 5 cm eine 150 Watt Quecksilberhochdruckdampflampe mit einem Quarzkühlmantel befand, wurden 100 g Essigsäure unter Rühren mittels eines Magnetrührers 6,5 h bei 90°C bestrahlt. Die gesamte Anordnung war, um Lichtverluste zu vermeiden, mit einer reflektierenden Folie umgeben. Während der Bestrahlung wurde kontinuierlich mit einem Gemisch aus Schwefeldioxid und Luft (10 NL/h SO₂, 10 NL/h Luft) begast. Die Aufarbeitung erfolgte wie oben beschrieben.

Der Austrag betrug 6 g mit einem Gewichtsanteil von 31 % MSS =̂ 1,86 g MSS =̂ 1,9 g/kWh MSS.

### Beispiel 5

In einem senkrecht angeordneten rohrförmigen Druckreaktor (Länge 18 cm, Innendurchmesser 8 cm) mit konzentrisch angeordnetem innenliegendem, druckfestem Quarzglasrohr (Außendurchmesser 4 cm, Wandstärke 5 mm), welches sowohl am oberen und unteren Deckel druckdicht in der Art und Weise anschließt, daß durch eine entsprechende Bohrung des Deckels eine Lichtquelle in das Innere des Rohrs eingebracht werden kann, wird im Gleichstrom von unten nach oben Essigsäure sowie über eine konzentrisch angeordnete Ringdüse ein Gasgemisch aus Schwefeldioxid (40 Norm-L/h) und Luft (100 Norm-L/h) unter Druck eingeleitet. Das den Reaktor verlassende Gemisch wird entspannt und die flüssigen Komponenten im Kreis geführt. In diesen Reaktor wurden aufsteigend 1100 g Essigsäure mit einem Durchfluß von 80 bis 100 L/h im Kreis gepumpt.
Das Reaktionsgemisch wurde bei 4 bar und 90°C 6,5 h lang mit einer 150 W-Quecksilberhochdruckdampflampe bestrahlt. Man erhielt 39 g Methansulfonsäure (40 g/kWh) bei einem Gewichtsverhältnis von Schwefelsäure zu Methansulfonsäure von 50/100.

### Beispiele 6 bis 8

In einem senkrecht angeordneten rohrförmigen Reaktor (Länge 25 cm, Innendurchmesser 15 cm) mit konzentrisch angeordnetem innenliegendem Quarzglasrohr (Außendruckmesser 6 cm), welches am oberen Deckel in der Art und Weise anschließt, daß durch eine entsprechende Öffnung im Deckel eine Quecksilberhochdruckdampflampe in das Innere des Rohrs eingebracht werden kann, wird im Gleichstrom von unten nach oben Essigsäure sowie über eine mittig am Reaktorboden angeordnete Glasringdüse bzw. Fritte Schwefeldioxid und ein Stickstoff/Sauerstoffgemisch eingeleitet. Die flüssigen Komponenten des Reaktoraustrags werden im Kreis geführt. In diesen Reaktor wurden aufsteigend 4200 g Essigsäure mit einem Durchfluß von 80 L/h bei 90°C im Kreis gepumpt. Das Reaktionsgemisch wurde bei Normaldruck und 90°C 6,5 h lang mit Quecksilberhochdrucklampen bestrahlt.

Quantenströme der Lampen (240 bis 320 nm) =
Die 150 W-Lampe hat einen Quantenstrom von 0,128 Mol Quanten/h.
Die 700 W-Lampe hat einen Quantenstrom von 0,6 Mol Quanten/h.

**Tabelle 2:**

| Reaktionsbedingungen und Versuchsergebnisse der Beispiele 6 bis 9 | | | | | | | | |
|---|---|---|---|---|---|---|---|---|
| Bsp | Eingasung | SO₂ [NL/h] | N₂/O₂ | | Lampe | MSS [g] | Gewichtsverhältnis H₂SO₄/MSS | Rate MSS [g/kWh] |
| 6 | Ringdüse | 40 | 8/2, | 40NL/h | 150 W | 48 | 39/100 | 71 |
| 7 | Glasfritte | 20 | 8/2, | 20NL/h | 700 W | 199 | 8,5/100 | 44 |
| 8 | Glasfritte | 40 | 8/2, | 160NL/h | 700 W | 399 | 23/100 | 88 |

## Patentansprüche

1. Verfahren zur Herstellung von Methansulfonsäure durch Bestrahlung einer Essigsäure, Schwefeldioxid und Sauerstoff enthaltenden Mischung, dadurch gekennzeichnet, daß die kumulierte Strahlungsdichte im Bereich von 240 bis 320 nm an der Lichteintrittsfläche des Lichts in die Reaktionsmischung im Mittel von 0,1 bis 50 mMol Quanten/cm²h beträgt.

2. Verfahren nach Anspruch 1, dadurch gekennzeichnet, daß man die Reaktion in einem Schlaufenreaktor durchführt.

3. Verfahren nach den Ansprüchen 1 oder 2, dadurch gekennzeichnet, daß man die Umsetzung bei einer Temperatur von 0 bis 160°C durchführt.

4. Verfahren nach den Ansprüchen 1 bis 3, dadurch gekennzeichnet, daß sich die Strahlungsquelle als Tauchlampe in der Reaktionsmischung befindet.

5. Verfahren nach den Ansprüchen 1 bis 4, dadurch gekennzeichnet, daß man der Reaktionsmischung Hilfsstoffe zusetzt.

6. Verfahren nach den Ansprüchen 1 bis 5, dadurch gekennzeichnet, daß man die Umsetzung bei einem Absolutdruck bis zu 20 bar durchführt.

## Claims

1. A process for preparing methanesulfonic acid by irradiating a mixture comprising acetic acid, sulfur dioxide and oxygen, wherein the cumulative irradiance in the range from 240 to 320 nm averages from 0.1 to 50 mmol quanta/cm²h at the area where the light enters the reaction mixture.

2. A process as claimed in claim 1, wherein the reaction is carried out in a loop reactor.

3. A process as claimed in claim 1 or 2, wherein the reaction is carried out at from 0 to 160°C.

4. A process as claimed in any of claims 1 to 3, wherein the radiation source is a lamp immersed in the reaction mixture.

5. A process as claimed in any of claims 1 to 4, wherein auxiliaries are added to the reaction mixture.

6. A process as claimed in any of claims 1 to 5, wherein the reaction is carried out under an absolute pressure of up to 20 bar.

## Revendications

1. Procédé pour la préparation d'acide méthanesulfonique en exposant à l'action de rayons un mélange comprenant de l'acide acétique, du dioxyde de soufre et de l'oxygène, caractérisé en ce que la densité cumulée du rayonnement dans le domaine de 240 à 320 nm à la surface d'incidence de la lumière dans le mélange réactionnel s'élève en moyenne de 0,1 à 50 mmoles de quanta/cm²h.

2. Procédé selon la revendication 1, caractérisé en ce qu'on effectue la réaction dans un réacteur en boucle.

3. Procédé selon les revendications 1 ou 2, caractérisé en ce qu'on effectue la mise en réaction à une température de 0 à 160°C.

4. Procédé selon les revendications 1 à 3, caractérisé en ce que la source de rayonnement se trouve dans le mélange réactionnel sous la forme d'une lampe à immersion.

5. Procédé selon les revendications 1 à 4, caractérisé en ce qu'on ajoute des adjuvants au mélange réactionnel.

6. Procédé selon les revendications 1 à 5, caractérisé en ce qu'on effectue la mise en réaction sous une pression absolue allant jusque 20 bar.
